# EUROPÄISCHE PATENTSCHRIFT

(11) **EP 1 020 415 B1**
(45) Veröffentlichungstag und Bekanntmachung des Hinweises auf die Patenterteilung: **04.06.2003**
(21) Anmeldenummer: 99117708.0
(22) Anmeldetag: 08.09.1999
(51) Int. Cl.: C04B 28/02, C04B 18/04

(54) **Verfahren zum Verfestigen schadstoffhaltiger, kontaminierter staubförmiger bis grobkörniger Anfallstoffe**
Process for solidifying dusty to coarse-grained contaminated noxious waste
Procédé pour la solidification de déchets nocifs contaminés poussiéreux ou à gros grains

(30) Priorität: 25.09.1998 DE 19844087; 06.11.1998 DE 19851256
(43) Veröffentlichungstag der Anmeldung: 19.07.2000
(73) Patentinhaber: FITR Gesellschaft für Innovation im Tief- und Rohrleitungsbau Weimar m.b.H., 99427 Weimar (DE)
(72) Erfinder: Berger, Wolfgang, Dr., 99423 Weimar (DE); Büchner, Ute, 99425 Weimar (DE); Krausewald, Jürgen, 99425 Weimar (DE)
(74) Vertreter: Kruspig, Volkmar, Dipl.-Ing.

(56) Entgegenhaltungen:
- EP-A- 0 467 483
- DE-A- 4 322 331
- DE-A- 4 441 835
- DE-C- 3 642 975
- FR-A- 2 545 387
- US-A- 5 502 267
- CHEMICAL ABSTRACTS, vol. 107, no. 26, 28. Dezember 1987 (1987-12-28) Columbus, Ohio, US; abstract no. 241615n, T. TSUBAHARA, ET AL: Seite 310; XP000016543 & JP 62 216951 A (ID.)

## Beschreibung

Die Erfindung betrifft ein Verfahren zum Verfestigen von insbesondere schadstoffhaltigen, kontaminierten staubförmigen bis grobkörnigen Anfallstoffen, Erdstoffen oder dergleichen Materialien unter Verwendung von Tonmineralien und hydraulischen Bindemitteln.

Es ist bekannt, daß in Böden eindringende Schadstoffe, insbesondere dann, wenn diese sandig und flüssigkeitsdurchlässig sind, das Grundwasser verunreinigen und somit zu einer Gefährdung der Trinkwasserversorgung führen können. Gegebene technische und wirtschaftliche Nachteile bisher bekannter Sanierungsmaßnahmen, wie z.B. das Abtragen von Bodenschichten, das Absenken des Grundwassers durch Einbringen von Barrieren in den Boden und/oder Abpumpen der Schadstofflösungen sowie die hier gegebenen Risiken haben zu Methoden geführt, bei denen vom Grundgedanken der Immobilisierung der Schadstoffe ausgegangen wird.

Zu den bekannten Immobilisierungs-Methoden gehört die Mikroeinkapselung, wobei hier eine Injektion von Verfestigungs- und Abdichtungsmitteln in den Schadstoffbereich erfolgt, um die Schadstoffe vor Ort zu binden. Das Umschließen eines Schadstoffbereichs mit einer bentonit- oder tonhaltigen vertikalen Abdichtung in Form einer Dichtwand wird als Makroeinkapselung bezeichnet.

Aus der PCT WO 83/01204 ist ein Verfahren zur Lagerung von organisch belasteten Mineralien bekannt, welche zur Adsorption in einem organophilen Bentonit eingebunden werden. Nachteilig ist, daß dort der gesamte Müllkörper mit organischem Bentonit versetzt oder Sickerwasser durch einen Filterkörper aus organophilem Bentonit gepumpt werden muß. Zur Deponieabdeckung muß demnach eine Lage aus reinem organophilen Bentonit ausgebildet werden.

Aus der DE 43 22 331 A1 ist ein Verfahren zum Immobilisieren von in Mauerwerken enthaltenen Schadstoffen anorganischer oder organischer Natur mittels eines Einbindungsmittels, bestehend aus einem Tonmineralgemisch, Zement und/oder Flugasche vorbekannt. Gemäß der dortigen Aufgabenstellung sollen die mit Schadstoffen belasteten Materialien zu einem Abfallkörper ausgebildet werden, wobei dieser Körper selbst als Barriere wirksam wird und daher auf Deponien endgelagert werden kann.

Das nach DE 43 22 331 A1 verwendete Einbindungsmittel besteht aus einem Tonmineralgemisch, Zement und/oder Flugasche, wobei das zu behandelnde, einen Gehalt von bis zu 60 M % an Tonmineralien aufweisende Material mit einem Anteil an Einbindungsmittel von 1 bis 60 M %, bezogen auf das Material und einen für die hydraulische Bindung erforderlichen Wasseranteil gemischt und in die Form eines dichten Bindemittel-Gesteingefüges gebracht wird. Hierfür muß das zu behandelnde Material vorab zerkleinert werden.

Es hat sich jedoch gezeigt, daß enthaltene Schadstoffe verschiedener Natur nicht ausreichend gebunden werden können, wodurch die Gefahr besteht, daß bei der Endlagerung unter Wasserzufuhr entsprechende Schadstoffkomponenten herausgelöst werden und in das Grundwasser eindringen. Es wird zwar mit der bekannten Lösung durch die Bentonit-Anteile und das hierdurch gegebene Quellvermögen das Bindemittel-Gesteingefüge als Barriere gegenüber Drittschadstoffen wirksam, die Gefahr des Austretens von Schadstoffen aus dem Gesteingefüge selbst kann jedoch, wie dargelegt, nicht ausgeschlossen werden.

Gemäß der DE 36 42 975 C1 wird ein Verfahren zur Herstellung eines zur Endlagerung tritiumhaltiger Abwässer geeigneten Festprodukts offenbart. Konkret wird aktivierter quellfähiger Bentonit mit tritiumhaltigem Wasser dispergierend vermischt und die daraus erhaltene Suspension einem Quellvorgang unterworfen. Die Supsension wird anschließend mit Zement dispergierend vermischt. Auf diese Weise wird das tritiumhaltige Material sicher eingeschlossen und ein Austreten des Schadstoffs kann wirksam verhindert werden.

Es ist unter Berücksichtigung des geschilderten Standes der Technik Aufgabe der Erfindung, ein weiterentwickeltes Verfahren zum Verfestigen von insbesondere schadstoffhaltigen, kontaminierten staubförmigen bis grobkörnigen Anfallstoffen anzugeben, das es gestattet, bei einem vertretbaren Aufwand von einzusetzenden Tonmineralien die Komponenten bereits in einer frühen Phase ausreichend und sicher zu binden, wodurch es möglich wird, verfestigte Gebilde einer unbedenklichen weiteren Verwendung zuzuführen.

Die Lösung der Aufgabe der Erfindung erfolgt mit einem Verfahren gemäß Definition nach der Lehre des Patentanspruchs 1, wobei die Unteransprüche zweckmäßige Ausgestaltungen und Weiterbildungen umfassen. Das Verfahren ist auch für nichtkontaminierte Lockergesteine oder recycelte Materialien wie keramische und/oder mineralisch gebundene Baustoffe oder recycelte Straßenbaumaterialien verwendbar.
Erfindungsgemäß wird verfahrensseitig zunächst eine wässrige Suspension enthaltend im wesentlichen 50 bis 200g Tonmehl auf 1 Liter Wasser in einem intensiven Mischprozeß gebildet, wodurch sich ein Energieeintrag einstellt, welcher das Quellverhalten unterstützt und die Schichtzwischenräume der Tonminerale der Suspension vergrößert.

Durch das Vermischen der staubförmigen bis grobkörnigen Anfallstoffe mit der oben genannten Suspension können dann Schadstoffe bzw. Schadstoffkomponenten in die Schichtzwischenräume der Tonminerale aufgenommen bzw. eingelagert werden, wodurch der gewünschte Adsorptions- und Bindungseffekt eintritt.

In einem weiteren Verfahrensschritt wird hydraulisches Bindemittel zum Verfestigen der Mischung dem Anfallstoff, der Suspension oder der Mischung aus beidem zugegeben. Die Zugabe des anorganischen Bindemittels bewirkt demnach sowohl die Verfestigung der Anfallstoff-Tonmehl-Bindemittel-Wasser-Mischung als auch dadurch bedingt das Einbinden der Schadstoffe In die gegebene Matrix.

In Abhängigkeit von den Eigenschaften bzw. der Zusammensetzung des oder der Anfallstoffe wird diesen eine Menge von 10 bis 40 M % Tonmehlsuspension und eine Menge von 1 bis 10 M % anorganisches Bindemittel jeweils bezogen auf den Anfallstoff zugegeben. Bevorzugt wird als Tonmehl aktivierter Bentonit eingesetzt. Bentonit ist ein Ton, der neben wenig Quarz überwiegend das Tonmineral Montmorillonit enthält und der aus der Verwitterung vulkanischer Aschen oder junger, heller, größtenteils glasig erstarrter Erdgußgesteine entstanden ist.

Eine weitere Verbesserung des Einbinde- und Anlagerungsprozesses von Schadstoffkomponenten wird durch Zugabe von weiterem Tonmehl als Trockenkomponente zur Tonmehlsuspension in einer Menge von bis 50 M % bezogen auf den Anfallstoff erreicht.

Es sei an dieser Stelle angemerkt, daß innerhalb der angegebenen Bereichsgrenzen der Zugabe von Tonmehl bzw. von hydraulischem Bindemittel die 20 Endfestigkeiten je nach dem sich anschließenden Verwendungszweck oder der Art der Endlagerung eingestellt werden können, ohne daßsich die vorteilhaften Eigenschaften bezüglich des Einbindens der Schadstoffe nachteilig verändern.

Als anorganisches hydraulisches Bindemittel wird bevorzugt auf Zement oder Flugasche bzw. aus einem Gemisch dieser zurückgegriffen.

In einer Ausführungsform des Verfahrens wird bezogen auf das kontaminierte Ausgangsmaterial, d.h. den Anfallstoff, eine Menge von 1 bis 10 M % anorganisches Bindemittel dem Gemisch aus Anfallstoff/Tonmehlsuspension zugegeben, wobei sich die Tonminerale mit den angelagerten Schadstoffen in die Bindemittelmatrix einbinden und anschließend ein Aushärten des Gemisches aus Tonmehl, Anfallstoff, anorganischem Bindemittel und Wasser erfolgt.

Um das Aus- bzw. Erhärtungsverhalten steuern zu können, besteht die Möglichkeit, dem Anfallstoff-Tonmehl-Bindemittel-Gemisch Abbindebeschleuniger oder -verzögerer zuzugeben. Entsprechende Abbindebeschleuniger oder Erstarrungsverzögerer sind aus der Betontechnologie bekannt.

Eine spezielle Verwendung des erfindungsgemäßen Verfahrens besteht im Verfestigen von unkontaminiertem Lockergestein natürlichen Ursprungs, wie Schluff, Sand, Kies oder dergleichen Materialien, aber auch von recyceltem Material, sogenanntem RC-Sand im Korngrößenbereich von 0 bis 100 mm.

Die Erfindung soll nachstehend anhand eines Ausführungsbeispiels näher erläutert werden.

Beim Ausführungsbeispiel wurde von einem kontaminierten RC-Material vorliegend im Korngemisch 0-8 mm ausgegangen.

Das recycelte Material kann auf eine bestimmte Korngröße verbrachtes Bauschuttmaterial, aber auch Material aus dem Straßenbausektor, z.B. Fahrbahnaltbeläge, welche Teer und andere Schadstoffe enthalten, umfassen.

Die durch intensiven Mischprozeß z.B. mit einem Kolloidial-Mischer gebildete Bentonitsuspension entstand durch Zugabe von 60 g Aktivbentonit auf 1 Liter Wasser.

Eine entsprechende Menge des RC-Materials wurde mit 16 M % der Tonmehlsuspension einem weiteren Mischprozeß unterzogen. Bei mindestens einem der beiden Mischprozesse erfolgt eine Zugabe von Bindemittel, z.B. gemäß Ausführungsbeispiel 2 M % Portlandzement CEM I 52,5 R.

Die Bentonitsuspension führte zu einem Absorbieren der Schadstoffe und einem Einbinden dieser, wobei die Portlandzement-Zugabe eine weitere Fixierung der Schadstoffe in der Matrix sowie die gewünschte Verfestigung der Anfallstoff-Tonmehl-Bindemittel-Wasser-Mischung gewährleistet.

Die Beritonitsuspension führte zu einem Absorbieren der Schadstoffe und einem Einbinden dieser, wobei die Portlandzement-Zugabe eine weitere Fixierung der Schadstoffe in der Matrix sowie die'gewünschte.'Verfestigung der Arifallstoff-Tonmehl-Bindemittel-Wasser-Mischung gewährleistet.

## Patentansprüche

1. Verfahren zum Verfestigen von insbesondere schadstoffhaitigen, kontaminierten staubförmigen bis grobkörnigen Anfallstoffen, Erdstoffen, Lockergestein oder recycelten Baumaterialien, unter Verwendung von Tonmineralien und hydraulischen Bindemitteln,
mit folgenden Schritten:
- Bilden einer wässrigen Suspension bestehend aus 50 bis 200 g Tonmehl je 1 l Wasser durch einen Intensiv-Mischprozeß zum Vergrößern der Schichtzwischenräume des Tonminerals,
- Vermischen der oben genannten Stoffe mit der vorbereiteten Suspension;
- Aushärten und Verfestigen der erhaltenen Mischung durch anorganisches Bindemittel,
wobei den oben genannten Stoffen 10 bis 40 M % Tonmehlsuspension und 1 bis 20 M % anorganisches Bindemittel jeweils bezogen auf den jeweiligen Anfallstoff zugegeben werden.

2. Verfahren nach Anspruch 1,
**dadurch gekennzeichnet, daß**
für die Suspensionsherstellung als Tonmehl aktivierter Bentonit eingesetzt wird.

3. Verfahren nach einem der vorangegangenen Ansprüche,
**dadurch gekennzeichnet, daß**
der Suspension zusätzlich anorganische Bindemittel zugegeben oder diese während des Intensiv-Mischprozesses zugeführt werden.

4. Verfahren nach einem der vorangegangenen Ansprüche,
**dadurch gekennzeichnet, daß**
als anorganisches hydraulisches Bindemittel Zement und/oder Flugasche eingesetzt wird.

5. Verfahren nach einem der vorangegangenen Ansprüche,
**dadurch gekennzeichnet, daß**
dem Gemisch aus Anfallstoff-Tonmehl-Bindemittel Abbindebeschleuniger oder - verzögerer zur Steuerung des Aushärtevorgangs zugegeben wird.

## Claims

1. A method for the compaction in particular of pollutant-containing, contaminated, powdery to coarse-grained excavated materials, earth materials, soil, or recycled construction materials, using clay minerals and hydraulic binders, comprising the following steps:
- preparing an aqueous suspension consisting of 50 to 200 g clay powder per 1 I water by means of an intensive mixing process for enlarging the layer interspaces of the clay mineral,
- mixing the above mentioned substances with the prepared suspension;
- setting and compacting the obtained mixture by means of an inorganic binder, with 10 to 40 mass percent of clay powder suspension and 1 to 20 mass percent of inorganic binder, each related to the respective excavated material, being added to the above-mentioned substances.

2. The method according to Claim 1,
**characterised in that**
as the clay powder activated bentonite is employed for preparing the suspension.

3. The method according to one of the previous claims,
**characterised in that**
inorganic binders are additionally added to the suspension or added during the intensive mixing process.

4. The method according to one of the previous claims,
**characterised in that**
cement and/or pulverised fuel ash is/are employed as an inorganic hydraulic binder.

5. The method according to one of the previous claims,
**characterised in that**
a curing accelerator or decelerator is added to the mixture of excavated material, clay powder, binder for controlling the setting process.

## Revendications

1. Procédé pour la solidification de matériaux de production, de matériaux terreux, de matières pierreuses en vrac ou de matériaux de construction recyclés, en particulier contenant des substances nocives, contaminées, et dont la consistance va de l'état poudreux à l'état à gros grains, en utilisant des minéraux argileux et des liants hydrauliques, comprenant les étapes suivantes :
- formation d'une suspension aqueuse se composant de 50 à 200 g de poudre d'argile pour 1 litre d'eau par un processus de mélange intensif pour agrandir les interstices entre les couches du minéral argileux ;
- mélange des substances précitées avec la suspension préparée ;
- durcissement et solidification du mélange obtenu au moyen de liants inorganiques,
dans lequel on ajoute 10 à 40% de la masse en suspension de minéral argileux et 1 à 20% de la masse en liants inorganiques aux substances précitées, en se référant respectivement au matériau de production respectif.

2. Procédé selon la revendication 1, **caractérisé par** l'utilisation de la bentonite activée à titre de poudre d'argile pour la préparation de la suspension.

3. Procédé selon l'une des revendications précédentes, **caractérisé par** l'ajout supplémentaire de liants inorganiques à la suspension ou pendant le processus de mélange intensif.

4. Procédé selon l'une des revendications précédentes, **caractérisé par** l'utilisation de ciment et/ou de cendres volantes à titre de liant hydraulique inorganique.

5. Procédé selon l'une des revendications précédentes, **caractérisé par** l'ajout d'accélérateurs ou de retardateurs de prise au mélange de liant; de poudre d'argile et de matériau de production, pour influencer sur le processus de durcissement.
